(19) 

**Europäisches Patentamt**

**European Patent Office**

**Office européen des brevets**

(11)  **EP 4 707 271 A1**

(12) 

# EUROPEAN PATENT APPLICATION
published in accordance with Art. 153(4) EPC

(43) Date of publication:
**11.03.2026 Bulletin 2026/11**

(21) Application number: **24901105.7**

(22) Date of filing: **05.12.2024**

(51) International Patent Classification (IPC):
*C07C 51/377* (2006.01)      *C07C 57/04* (2006.01)
*B01J 8/00* (2006.01)      *B01J 8/02* (2006.01)

(52) Cooperative Patent Classification (CPC):
**B01J 8/00; B01J 8/02; C07C 51/377; C07C 57/04**

(86) International application number:
**PCT/KR2024/019883**

(87) International publication number:
**WO 2025/121919 (12.06.2025 Gazette 2025/24)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  07.12.2023  KR 20230176547
03.12.2024  KR 20240177830

(71) Applicant: **LG Chem, Ltd.**
**Seoul 07336 (KR)**

(72) Inventors:
• **JUNG, Dabin**
**Daejeon 34122 (KR)**
• **SHIN, Daeyoung**
**Daejeon 34122 (KR)**
• **KIM, Mi Kyung**
**Daejeon 34122 (KR)**

• **JEONG, Hoiin**
**Daejeon 34122 (KR)**
• **IM, Yong O**
**Daejeon 34122 (KR)**
• **LEE, Jiyoung**
**Daejeon 34122 (KR)**
• **KANG, Tae Hun**
**Daejeon 34122 (KR)**
• **KIM, Hyunji**
**Daejeon 34122 (KR)**
• **KIM, Seungik**
**Daejeon 34122 (KR)**
• **LEE, Jongheon**
**Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(54)  **METHOD AND APPARATUS FOR PRODUCING ACRYLIC ACID**

(57)  The present disclosure relates to a method and apparatus for producing acrylic acid. Specifically, the present disclosure relates to a method for producing acrylic acid, comprising a two-stage reactor that allows the acrylic acid yield to achieve a target level, and an apparatus therefor.

[FIG. 1]

$$L = L1 + L2$$

EP 4 707 271 A1

**Description**

**[TECHNICAL FIELD]**

CROSS-REFERENCE TO RELATED APPLICATION(S)

This application claims priority to and the benefit of Korean Patent Application No. 10-2023-0176547, filed on December 7, 2023, and Korean Patent Application No. 10-2024-0177830, filed on December 3, 2024, with the Korean Intellectual Property Office, the disclosures of which are incorporated herein by reference in their entirety.

The present disclosure relates to a method and apparatus for producing acrylic acid. Specifically, the present disclosure relates to a method for producing acrylic acid, comprising a two-stage reactor that allows the acrylic acid yield to achieve a target level, and an apparatus therefor.

**[BACKGROUND ART]**

Acrylic acid is an organic compound having both a carboxylic acid and an unsaturated double bond in its molecule, and is used in various industrial fields because it has an extremely simple structure and can be polymerized while being converted into various materials.

Specifically, acrylic acid can be used as polyacrylic acid, tacky-adhesives, paints, etc. required for the production of a superabsorbent polymer, or may be used as a raw material for the production of other types of acrylate-based monomers, or may also be used as a raw material for polymerization with various other monomers such as acrylamide, acrylonitrile, styrene, and alpha olefins.

This acrylic acid is generally produced by using propylene produced in the refining and separation process of crude oil, such as naphtha cracking.

However, recently, as concerns about the depletion of crude oil and environmental issues are increasing, interests in the method for producing acrylic acid using environmentally friendly raw materials are increasing.

Conventionally, in the method of producing acrylic acid through a gas-phase dehydration reaction of lactic acid on an acid catalyst, not only the production of acrylic acid which is a main reaction, but also numerous side reactions occur simultaneously. Therefore, it is necessary to design appropriate reaction conditions and reactors so that a desired level of acrylic acid yield can be obtained.

**[DETAILED DESCRIPTION OF THE INVENTION]**

**[Technical Problem]**

It is an object of the present disclosure to provide a method for producing acrylic acid, which comprises a two-stage reactor designed to achieve a desired yield of acrylic acid in the production of acrylic acid by a lactic acid dehydration reaction, and an apparatus therefor.

**[Technical Solution]**

According to the present disclosure, provided is an apparatus for producing acrylic acid, which comprises a two-stage reactor for lactic acid dehydration reaction, with the two-stage reactor comprising: a feed supply unit to which lactic acid gas is supplied; a first-stage dehydration reactor filled with a catalyst for lactic acid dehydration reaction; and a second-stage dehydration reactor filled with a catalyst for lactic acid dehydration reaction, wherein the apparatus satisfies the following Mathematical Formula 1:

[Mathematical Formula 1]

$$0.3 \leq \frac{L_1}{L_1 + L_2} \leq 0.6$$

wherein in Mathematical Formula 1, $L_1$ is the length of the first-stage dehydration reactor, and $L_2$ is the length of the second-stage dehydration reactor.

According to one embodiment, the yield of acrylic acid obtained from the apparatus for producing acrylic acid may be 53% or more.

According to one embodiment, the first-stage dehydration reactor and the second-stage dehydration reactor are connected by a transfer line, wherein the transfer line may further comprise a heating unit.

In addition, according to the present disclosure, there is provided a method for producing acrylic acid, the method comprising: a first step of supplying a feed stream containing lactic acid gas molecules from a feed supply unit to an upper end of a first-stage dehydration reactor filled with a catalyst, performing a dehydration reaction, and then discharging the stream to the lower end thereof; and a second step of supplying the stream discharged from the lower end of the first-stage dehydration reactor to an upper end of a second-stage dehydration reactor filled with a catalyst, performing a dehydration reaction, and then discharging the stream to the lower end, wherein the feed stream supplied to the upper end of the first-stage dehydration reactor contains lactic acid gas, and wherein the method satisfies the following Mathematical Formula 2:

[Mathematical Formula 2]

$$0.3 \leq \frac{T_1}{T_1 + T_2} \leq 0.6$$

wherein in Mathematical Formula 2, $T_1$ is the average time that the feed stream resides in the first-stage dehydration reactor, and $T_2$ is the average time that the stream supplied to the second-stage dehydration reactor resides in the second-stage dehydration reactor.

According to one embodiment, the method for producing acrylic acid may have an acrylic acid yield of 53 mol% or more.

According to one embodiment, the stream discharged from the lower end of the first-stage dehydration reactor is transferred to the second-stage dehydration reactor through a transfer line, and the temperature of the stream during transfer may be maintained at 370°C or more and 410°C or less.

As used herein, the terms "a first," "a second," etc. are used herein to explain various constitutional elements, and these terms are used only to distinguish one constitutional element from another constitutional element.

Further, the technical terms used herein is only to explain exemplary embodiments and is not intended to limit the scope of the present disclosure.

The singular forms "a," "an" and "the" are intended to include plural forms, unless the context clearly indicates otherwise.

It should be understood that the terms "comprise," "include", "have", etc. are used herein to specify the presence of stated features, integers, steps, components or combinations thereof, but do not preclude the presence or addition of one or more other features, integers, steps, components, or combinations thereof.

Also, as used herein, in case a layer or an element is mentioned to be formed "on" or "above" layers or elements, it means that the layer or element is directly formed on the layers or elements, or it means that other layers or elements may be additionally formed between the layers, on a subject, or on a substrate.

Although the present disclosure may have various forms and various modifications may be made thereto, specific examples will be exemplified and explained in detail below. However, it is not intended to limit the present disclosure to specific disclosure, and it should be understood that the present disclosure includes all the modifications, equivalents or replacements thereof without departing from the spirit and technical scope of the present disclosure.

In the gas-phase dehydration reaction of lactic acid, generally, vaporized lactic acid is added to a reactor filled with a catalyst to cause a dehydration reaction, and acrylic acid produced by the dehydration reaction is discharged from a lower end of the reactor.

In the process of producing acrylic acid through the gas-phase dehydration reaction of lactic acid, not only acrylic acid, which is a product of main reaction, but also by-products of numerous side reactions are produced. Therefore, it is necessary to design appropriate reaction conditions and reactors so that a desired level of acrylic acid yield can be obtained.

Thus, according to the present disclosure, provided is a method for producing acrylic acid, which comprises a two-stage reactor designed to achieve a desired yield of acrylic acid in the production of acrylic acid, and an apparatus therefor.

Specifically, according to the present disclosure, provided is a method for producing acrylic acid, comprising a two-stage reactor which controls the length of a catalyst stage included in the two-stage reactor or the average time that the stream resides in the reactor including the catalyst stage to obtain a desired level of acrylic acid yield, and an apparatus therefor.

The apparatus for producing acrylic acid according to the present disclosure will be discussed below.

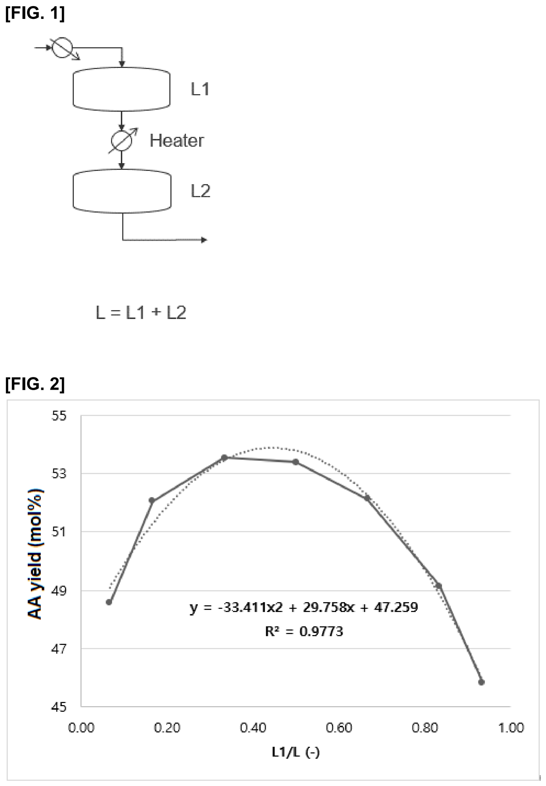

According to one aspect of the present disclosure, provided is an apparatus for producing acrylic acid, which comprises a two-stage reactor for lactic acid dehydration reaction, with the two-stage reactor comprising: a feed supply unit to which lactic acid gas is supplied; a first-stage dehydration reactor filled with a catalyst for a lactic acid dehydration reaction; and a second-stage dehydration reactor filled with a catalyst for a lactic acid dehydration reaction (see FIG. 1).

The apparatus for producing acrylic acid according to the present disclosure may satisfy the following Mathematical Formula 1.

[Mathematical Formula 1]

$$0.3 \leq \frac{L_1}{L_1 + L_2} \leq 0.6$$

In Mathematical Formula 1, $L_1$ is the length of the first-stage dehydration reactor, and $L_2$ is the length of the second-stage dehydration reactor.

That is, in the case of the apparatus for producing acrylic acid in which the first stage length relative to the total reaction stage length satisfies the range of 0.3 to 0.6, the yield of acrylic acid obtained from the production apparatus may be 53% or more. In this case, the yield of acrylic acid can be calculated by the following Mathematical Formula 3.

Acrylic acid yield (mol%) = (Number of moles of acrylic acid produced in the second-stage reactor / Number of moles of lactic acid added to the first-stage reactor) * 100          [Mathematical Formula 3]

The number of moles of acrylic acid produced in the second-stage reactor was measured by measuring the number of moles of acrylic acid in the discharge stream of the second-stage reactor, and the number of moles of lactic acid added to the first-stage reactor by measuring as the number of moles of lactic acid in the feed stream added to the first-stage reactor.

If the desired acrylic acid yield being set to 53 mol% or more, the yield of the acrylic acid is the highest in the current process. As the yield of acrylic acid is higher, the consumption unit is lower, whereby as the yield of acrylic acid is higher, it is better. Specifically, when the acrylic acid yield is increased from 50 mol% to 53 mol%, the consumption unit of lactic acid is decreased by about 5 to 6%. Since the consumption unit of lactic acid accounts for the largest proportion of the production cost of acrylic acid, improving the acrylic acid yield is very important for reducing the cost. At this time, the consumption unit of lactic acid is the value obtained by dividing the total amount (kg) of lactic acid added to the process by the amount (kg) of acrylic acid produced, and as the consumption unit of lactic acid is lower, the process is more economical.

The first-stage dehydration reactor and the second-stage dehydration reactor can be sequentially arranged up and down.

The upper end of the first-stage dehydration reactor is connected to a feed supply unit to which lactic acid gas is supplied. Further, the dehydration reaction catalyst filled in the first-stage dehydration reactor may include at least one selected from the group consisting of a calcium phosphate-based catalyst, a sodium phosphate-based catalyst, and an aluminum phosphate-based catalyst, and the other reaction conditions may be used without particular limitation as long as they are generally used in the technical field to which the present disclosure pertains, unless they are contrary to the contents defined herein.

More specifically, the dehydration catalyst may include $CaSO_4/Na_2SO_4$; $Na_4P_2O_7/CaSO_4$; $Na_4P_2O_7/Ca_3(PO_4)_2$; $NaH_2PO_4$-$NaHCO_3/SiO_2$; $AlPO_4$-$NH_3$; $Ca_3(PO_4)_2/CaSO_4$; $Ca_2P_2O_7$; $Ca_5(PO_4)_3(OH)$, and the like.

The lower end of the first-stage dehydration reactor may include a transfer line that is connected to the upper end of the second-stage dehydration reactor. Further, the transfer line may include a heating unit. The lactic acid dehydration reaction is a reaction that is performed at a high temperature. Thus, when the temperature of the stream discharged from the first-stage dehydration reactor is lowered in the process of transferring it to the second-stage dehydration reactor, heat loss for the dehydration reaction occurs by that extent, so the transfer line may include a heating unit in order to increase the temperature of the discharged stream to the dehydration reaction temperature.

As an example, the temperature of the stream discharged from the first-stage reactor may be about 350 to 360°C, and the stream may pass through the transfer line and be heated to about 390°C by the heating unit, and then added to the two-stage reactor.

The upper end of the second-stage dehydration reactor is connected to a transfer line that transfers the stream discharged from the first-stage dehydration reactor. Further, the dehydration reaction catalyst filled in the second-stage dehydration reactor may include at least one selected from the group consisting of a calcium phosphate-based catalyst, a sodium phosphate-based catalyst, and an aluminum phosphate-based catalyst, and the other reaction conditions may be used without particular limitations as long as they are generally used in the technical field to which the present disclosure pertains, unless they are contrary to the contents defined herein.

More specifically, the dehydration catalyst may include $CaSO_4/Na_2SO_4$; $Na_4P_2O_7/CaSO_4$; $Na_4P_2O_7/Ca_3(PO_4)_2$; $NaH_2PO_4$-$NaHCO_3/SiO_2$; $AlPO_4$-$NH_3$; $Ca_3(PO_4)_2/CaSO_4$; $Ca_2P_2O_7$; $Ca_5(PO_4)_3(OH)$, and the like.

In the second-stage dehydration reactor, the product stream is discharged from the lower end after a further dehydration reaction has been performed. Thus, a discharge line may be connected to the lower end of the second-stage dehydration reactor.

In addition, the product stream discharged from the second-stage dehydration reactor can be moved to a condenser along the discharge line. In the condenser, the acrylic acid contained in the product stream in a gaseous state can be condensed, liquefied, and collected.

Next, the method for producing acrylic acid according to the present disclosure will be discussed step by step.

**[First step]**

The first step of the present disclosure is a step of supplying a feed stream from a feed supply unit to an upper end of a first-stage dehydration reactor filled with a catalyst, performing a dehydration reaction, and then discharging the stream to the lower end thereof.

The feed stream supplied to an upper end of the first-stage dehydration reactor may include vaporized lactic acid gas molecules. As an example, the vaporized lactic acid molecules may be obtained by supplying a stream containing lactic acid to the vaporization reactor and performing a vaporization reaction of lactic acid in the inside of the vaporization reactor.

The catalyst filled in the first-stage dehydration reactor is a catalyst for lactic acid dehydration reaction, and may include at least one selected from the group consisting of a calcium phosphate-based catalyst, a sodium phosphate-based catalyst, and an aluminum phosphate-based catalyst, and the other reaction conditions may be used without particular limitation as long as they are generally used in the technical field to which the present disclosure pertains, unless they are contrary to the contents defined herein.

More specifically, the dehydration catalyst may include $CaSO_4/Na_2SO_4$; $Na_4P_2O_7/CaSO_4$; $Na_4P_2O_7/Ca_3(PO_4)_2$; $NaH_2PO_4$-$NaHCO_3/SiO_2$; $AlPO_4$-$NH_3$; $Ca_3(PO_4)_2/CaSO_4$; $Ca_2P_2O_7$; $Ca_5(PO_4)_3(OH)$, and the like.

The average dehydration reaction temperature of the first-stage dehydration reactor may be a temperature of more than 340°C and less than about 400°C, preferably a temperature of more than 340°C, or 345°C or more, or 350°C or more, or about 355°C or more, and about 400°C or less, or about 390°C or less, or about 380°C or less.

If the dehydration reaction temperature is too low, there may be a problem that the lactic acid conversion rate and acrylic acid yield is significantly reduced. Conversely, if the dehydration reaction temperature is too high, i) an aldehyde production reaction by decarboxylation or decarbonylation, ii) a propanoic acid production reaction, etc. may be further promoted, which causes a problem that by-products are made high.

A stream which has finished the dehydration reaction may be discharged from the lower end of the first-stage dehydration reactor, and the stream discharged from the lower end of the first-stage dehydration reactor may be transferred to the second-stage dehydration reactor through a transfer line.

The stream discharged from a lower end of the first-stage dehydration reactor may include a gas-phase acrylic acid, unreacted lactic acid gas molecules and by-products (e.g., acetaldehyde, propionic acid, 2,3-pentanedione, etc.) produced through the lactic acid dehydration reaction.

The transfer line is provided with a heating unit, whereby while the stream discharged from the first-stage dehydration reactor being transferred to the two-stage reactor through the transfer line, the temperature of the stream may be maintained at 370°C or more and 410°C or less. The heating unit may be a separate heating device, such as a heat exchanger or a heating band.

The lactic acid dehydration reaction is a reaction that is performed at a high temperature. Thus, when the temperature of the stream discharged from the first-stage dehydration reactor is lowered in the process of transferring it to the second-stage dehydration reactor, heat loss for the dehydration reaction occurs by that extent, so the transfer line may satisfy the above temperature range in order to increase the temperature of the discharged stream to the dehydration reaction temperature.

**[Second step]**

The second step of the present disclosure is a step of supplying a stream discharged from a lower end of the first-stage dehydration reactor to an upper end of a second-stage dehydration reactor filled with a catalyst, performing a dehydration reaction, and then discharging the stream to the lower end.

The stream discharged from the lower end of the first-stage dehydration reactor, which is supplied to an upper end of the second-stage dehydration reactor, may contain unreacted lactic acid gas molecules. Further, the stream discharged from a lower end of the first-stage dehydration reactor may be supplied to an upper end of the second-stage dehydration reactor through a transfer line.

The catalyst filled in the second-stage dehydration reactor is a catalyst for a lactic acid dehydration reaction, and may include at least one selected from the group consisting of a calcium phosphate-based catalyst, a sodium phosphate-based catalyst, and an aluminum phosphate-based catalyst, and the other reaction conditions may be used without particular limitation as long as they are generally used in the technical field to which the present disclosure pertains, unless they are contrary to the contents defined herein.

More specifically, the dehydration catalyst may include $CaSO_4/Na_2SO_4$; $Na_4P_2O_7/CaSO_4$; $Na_4P_2O_7/Ca_3(PO_4)_2$; $NaH_2PO_4$-$NaHCO_3/SiO_2$; $AlPO_4$-$NH_3$; $Ca_3(PO_4)_2/CaSO_4$; $Ca_2P_2O_7$; $Ca_5(PO_4)_3(OH)$, and the like.

The average dehydration reaction temperature of the second-stage dehydration reactor may be a temperature of more than 340°C and about 400°C or less, preferably a temperature of more than 340°C, or about 345°C or more, or about 350°C or more, or about 355°C or more, and about 400°C or less, or about 390°C or less, or about 380°C or less.

If the temperature of the dehydration reaction is too low, there may be a problem that the lactic acid conversion rate and the acrylic acid yield are significantly reduced. On the contrary, if the dehydration reaction temperature is too high, i) an aldehyde production reaction by decarboxylation or decarbonylation, ii) a propanoic acid production reaction, etc. may be further promoted, which causes a problem that by-products are made high.

In the second-stage dehydration reactor, an additional dehydration reaction is performed, and then the product stream is discharged to the lower end. Thus, a discharge line is connected to the lower end of the second-stage dehydration reactor to discharge the product stream.

In addition, the product stream discharged from the second-stage dehydration reactor can be moved to a condenser along the discharge line. In the condenser, acrylic acid contained in the product stream in a gaseous state can be condensed, liquefied, and collected.

Further, the method for producing acrylic acid according to the present disclosure can satisfy the following Mathematical Formula 2.

[Mathematical Formula 2]

$$0.3 \le \frac{T_1}{T_1 + T_2} \le 0.6$$

In Mathematical Formula 2, $T_1$ is the average time that the feed stream resides in the first-stage dehydration reactor, and $T_2$ is the average time that the stream supplied to the second-stage dehydration reactor resides in the second-stage dehydration reactor.

That is, in the case of the method for producing acrylic acid in which the time that the feed stream resides in the first-stage dehydration reactor relative to the total reaction time satisfies the range of 0.3 or more to 0.6 or less, the yield of acrylic acid obtained from the production method may be 53% or more. In this case, the yield of acrylic acid can be calculated by the following Mathematical Formula 3.

Acrylic acid yield (mol%) = (Number of moles of acrylic acid produced in the second-stage reactor / Number of moles of lactic acid added to the first-stage reactor) * 100          [Mathematical Formula 3]

The number of moles of acrylic acid produced in the second-stage reactor can be measured by measuring the number of moles of acrylic acid in the discharge stream of the second-stage reactor, and the number of moles of lactic acid added to the first-stage reactor can be measured by measuring the number of moles of lactic acid in the feed stream added to the first-stage reactor.

If the desired acrylic acid yield being set to 53 mol% or more, the acrylic acid yield is the highest in the current process. As the yield of acrylic acid is higher, the consumption unit is lower, whereby as the yield of acrylic acid is higher, it is better. Specifically, when the acrylic acid yield is increased from 50 mol% to 53 mol%, the consumption unit of lactic acid is decreased by about 5 to 6%. Since the consumption unit of lactic acid accounts for the largest proportion of the production cost of acrylic acid, improving the acrylic acid yield is very important for reducing the cost. At this time, the consumption unit of lactic acid is the value obtained by dividing the total amount (kg) of lactic acid added to the process by the amount (kg) of acrylic acid produced, and as the consumption unit of lactic acid is lower, the process is more economical.

Further, in the case of the dehydration reaction using lactic acid gas, a fixed-bed cylindrical reactor is used, and the catalyst stage length of the internally filled catalyst of this fixed-bed cylindrical reactor is the same as the reactor length. Further, the first-stage reactor and the second-stage reactor have the same diameter, but the lengths of the catalyst stages, i.e., the lengths of the reactors, are different. That is, the first-stage and second-stage reactors for dehydration reaction of the present disclosure are respectively fixed-bed cylindrical reactors, and the time for which the stream resides in the first-stage or second-stage reactor is proportional to the length of each catalyst stage, i.e., the reactor.

## [ADVANTAGEOUS EFFECTS]

According to an embodiment of the present disclosure, by configuring the apparatus for producing acrylic acid with a two-stage reactor and controlling the length of the reaction stage, acrylic acid can be obtained in a target yield.

In addition, according to an embodiment of the present disclosure, by controlling the time of residence in the stream in the reactor using a two-stage reactor during the production of acrylic acid, acrylic acid can be obtained in a target yield.

## [BRIEF DESCRIPTION OF THE DRAWINGS]

FIG. 1 is a schematic diagram of an acrylic acid production device according to an embodiment of the present disclosure.

FIG. 2 is a graph showing the acrylic acid yield according to Formula 1 according to an embodiment of the present disclosure.

## [DETAILED DESCRIPTION OF THE EMBODIMENTS]

Hereinafter, the actions and effects of the invention will be described more specifically with reference to specific examples. However, these examples are presented only as the illustrations of the invention, and the scope of right of the invention is not determined thereby.

## [Preparation of gas-phase lactic acid feed stream]

A lactic acid aqueous solution with a concentration of 88 wt.% (product name: PURAC H888, available from Corbion) was prepared as a lactic acid raw material. The lactic acid raw material and distilled water were mixed in a weight ratio of 1:1.2 and refluxed under a temperature condition of about 95°C for about 18 hours to obtain a lactic acid aqueous solution with a concentration of about 40 wt.% in which lactic acid and lactic acid oligomers reached an equilibrium state, which was used as a feed for lactic acid vaporization. Nitrogen was used as a carrier gas, and the temperature inside the vaporization reactor was set to be 200°C to 300°C. The lactic acid molecular feed vaporized in the vaporization reactor was used as a feed for dehydration reaction.

## Experimental Example 1

### [Example 1]

The two-stage dehydration reactors for the dehydration reaction were fixed bed reactors, each of which was filled with a dehydration reaction catalyst. The length of the first-stage dehydration reactor was 1.5 m and the inner diameter was 4 m. The length and inner diameter of the second-stage dehydration reactor were configured to be the same as those of the first-stage dehydration reactor. Thereby, the value of Mathematical Formula 1 was 0.5. Hastelloy material was used for the first-stage and second-stage dehydration reactors. Further, the first-stage dehydration reactor and the second-stage dehydration reactor were respectively filled with a dehydration reaction catalyst.

A feed stream supply unit was provided at the upper end of the first-stage dehydration reactor, through which vaporized lactic acid molecule feed was supplied. A transfer line was provided between the first-stage dehydration reactor and the second-stage dehydration reactor, and the temperature was set so as to maintain a temperature of 390°C by a temperature regulator. The temperature regulator used was a shell and tube type heat exchanger. Further, a discharge line was provided at a lower end of the second-stage dehydration reactor to discharge the reaction product which had finished the dehydration reaction.

As the dehydration reaction catalyst, a composite calcium phosphate-based catalyst consisting of $Ca_2P_2O_7$ and $Ca_5(PO_4)_3(OH)$ formed into cylindrical pellets with a diameter of about 3 mm and a length of about 3 mm was used. At this time, the mixing weight ratio of $Ca_2P_2O_7$ and $Ca_5(PO_4)_3(OH)$ was 7:3.

A vaporized lactic acid feed stream having a temperature of 390°C was supplied to an upper end of the first-stage dehydration reactor. The residence time of the vaporized lactic acid feed stream inside the first-stage dehydration reactor was 0.8 seconds. At this time, the temperature drop in the first-stage dehydration reactor was 38.4°C, and the discharge temperature from the first-stage dehydration reactor was 351.6°C.

The stream which had finished the reaction in the first-stage dehydration reactor was transferred to the second-stage dehydration reactor through a transfer line. At this time, the temperature of the stream immediately discharged from the first-stage dehydration reactor was 351.6°C. Thus, the stream discharged from the first-stage dehydration reactor was heated in the transfer line connected to a lower end of the first-stage dehydration reactor, so that the temperature was set to be 390°C when introduced into the second-stage dehydration reactor.

At this time, the residence time of the stream transferred from the first-stage dehydration reactor inside the second-stage dehydration reactor was 0.75 seconds. Thereby, the value of Mathematical Formula 2 was 0.51.

The product stream which has finished the dehydration reaction in the second-stage dehydration reactor was discharged through the discharge line, and the temperature was 368.8°C.

The product stream discharged from the second-stage dehydration reactor was collected and quantitatively analyzed by high-performance liquid chromatography (HPLC), and the acrylic acid yield and the lactic acid conversion rate were calculated.

High performance liquid chromatography was analyzed by the following method.

The obtained reaction product was diluted 20 times in volume base using distilled water, and then quantitatively analyzed by HPLC using Agilent 1260 Infinity **II,** and HPLC analysis conditions were as follows.

- Eluent: 0.005 mol $H_2SO_4$ (aq)
- Eluent flow rate: 0.4 mL/min
- Column: Aminex HPX-87H
- Column temperature: 10°C
- Detector: UV 210-300 nm
- Analysis time: 70 min
- Analysis pressure: ~70 bar

The acrylic acid yield was calculated according to the following Mathematical Formula 3.

Acrylic acid yield (mol%) = (Number of moles of acrylic acid produced in the second-stage reactor / Number of moles of lactic acid added to the first-stage reactor) * 100   [Mathematical Formula 3]

The number of moles of acrylic acid produced in the second-stage reactor are measured by the number of moles of acrylic acid in the discharge stream of the second-stage reactor, and the number of moles of lactic acid added to the first-stage reactor are measured by the number of moles of lactic acid in the feed stream added to the first-stage reactor.

The lactic acid conversion rate was calculated according to the following Mathematical Formula 4.

Lactic acid conversion rate (%) = {Lactic acid addition amount (kg/hr) - Lactic acid flow rate (kg/hr) at an outlet of the two-stage reactor)} / Lactic acid addition amount (kg/hr) * 100   [Mathematical Formula 4]

[Example 2]

The two-stage dehydration reactors for a dehydration reaction were fixed bed reactors, each of which was filled with a dehydration reaction catalyst. The length of the first-stage dehydration reactor was 1 m and the inner diameter was 4 m. The length of the second-stage dehydration reactor was 2 m and the inner diameter was 4 m. Thereby, the value of Mathematical Formula 1 was 0.33. Hastelloy material was used for the first-stage and second-stage dehydration reactors. Further, the first-stage dehydration reactor and the second-stage dehydration reactor were respectively filled with a dehydration reaction catalyst.

A feed stream supply unit was provided at the upper end of the first-stage dehydration reactor, through which vaporized lactic acid molecule feed was supplied. A transfer line was provided between the first-stage dehydration reactor and the second-stage dehydration reactor, and the temperature was set so as to maintain a temperature of 390°C by a temperature regulator. The temperature regulator used was a shell and tube type heat exchanger. Further, a discharge line was provided at a lower end of the second-stage dehydration reactor to discharge the reaction product which had finished the dehydration reaction.

As the dehydration reaction catalyst, a composite calcium phosphate-based catalyst consisting of $Ca_2P_2O_7$ and $Ca_5(PO_4)_3(OH)$ formed into cylindrical pellets with a diameter of about 3 mm and a length of about 3 mm was used. At this time, the mixing weight ratio of $Ca_2P_2O_7$ and $Ca_5(PO_4)_3(OH)$ was 7:3.

A vaporized lactic acid feed stream having a temperature of 390°C was supplied to an upper end of the first-stage dehydration reactor. The residence time of the vaporized lactic acid feed stream inside the first-stage dehydration reactor was 0.5 seconds. At this time, the temperature drop inside the first-stage dehydration reactor was 33°C, and the discharge temperature from the first-stage dehydration reactor was 357°C.

The stream which had finished the reaction in the first-stage dehydration reactor was transferred to the second-stage dehydration reactor through a transfer line. At this time, the temperature of the stream immediately discharged from the first-stage dehydration reactor was 357°C. Thus, the stream discharged from the first-stage dehydration reactor was heated in the transfer line connected to a lower end of the first-stage dehydration reactor, so that the temperature was set to be 390°C when introduced into the second-stage dehydration reactor

At this time, the residence time of the stream transferred from the first-stage dehydration reactor inside the second-stage dehydration reactor was 1 second. Thereby, the value of Mathematical Formula 2 was 0.33.

The product stream which had finished the dehydration reaction in the second-stage dehydration reactor was discharged through the discharge line, and the temperature was 363°C.

The product stream discharged from the second-stage dehydration reactor was collected and quantitatively analyzed by high-performance liquid chromatography (HPLC), and the acrylic acid yield and the lactic acid conversion rate were calculated in the same manner as in Example 1.

**[Example 3]**

The two-stage dehydration reactors for a dehydration reaction were fixed bed reactors, each of which was filled with a dehydration reaction catalyst. The length of the first-stage dehydration reactor was 1.7 m and the inner diameter was 4 m. The length of the second-stage dehydration reactor was 1.3 m and the inner diameter was 4 m. Thereby, the value of Mathematical Formula 1 was 0.57. Hastelloy material was used for the first-stage and second-stage dehydration reactors. Further, the first-stage dehydration reactor and the second-stage dehydration reactor were respectively filled with a dehydration reaction catalyst.

A feed stream supply unit was provided at the upper end of the first-stage dehydration reactor, through which vaporized lactic acid molecule feed was supplied. A transfer line was provided between the first-stage dehydration reactor and the second-stage dehydration reactor, and the temperature was set so as to maintain a temperature of 390°C by a temperature regulator. The temperature regulator used was a shell and tube type heat exchanger. Further, a discharge line was provided at a lower end of the second-stage dehydration reactor to discharge the reaction product which has finished the dehydration reaction.

As the dehydration reaction catalyst, a composite calcium phosphate-based catalyst consisting of $Ca_2P_2O_7$ and $Ca_5(PO_4)_3(OH)$ formed into cylindrical pellets with a diameter of about 3 mm and a length of about 3 mm was used. At this time, the mixing weight ratio of $Ca_2P_2O_7$ and $Ca_5(PO_4)_3(OH)$ was 7:3.

A vaporized lactic acid feed stream having a temperature of 390°C was supplied to an upper end of the first-stage dehydration reactor. The residence time of the vaporized lactic acid feed stream inside the first-stage dehydration reactor was 0.9 seconds. At this time, the temperature drop in the first-stage dehydration reactor was 40°C, and the discharge temperature from the first-stage dehydration reactor was 350°C.

The stream which had finished the reaction in the first-stage dehydration reactor was transferred to the second-stage dehydration reactor through a transfer line. At this time, the temperature of the stream immediately discharged from the first-stage dehydration reactor was 350°C. Thus, the stream discharged from the first-stage dehydration reactor was heated in the transfer line connected to a lower end of the first-stage dehydration reactor, so that the temperature was set to be 390°C when introduced into the second-stage dehydration reactor.

At this time, the residence time of the stream transferred from the first-stage dehydration reactor inside the second-stage dehydration reactor was 0.64 second. Thereby, the value of Mathematical Formula 2 was 0.58.

The product stream which had finished the dehydration reaction in the second-stage dehydration reactor was discharged through the discharge line, and the temperature was 371°C.

The product stream discharged from the second-stage dehydration reactor was collected and quantitatively analyzed by high-performance liquid chromatography (HPLC), and the acrylic acid yield and the lactic acid conversion rate were calculated in the same manner as in Example 1.

**[Comparative Example 1]**

The two-stage dehydration reactors for a dehydration reaction were fixed bed reactors, each of which was filled with a dehydration reaction catalyst. The length of the first-stage dehydration reactor was 0.2 m and the inner diameter was 4 m. The length of the second-stage dehydration reactor was 2.8 m and the inner diameter was 4 m. Thereby, the value of Mathematical Formula 1 was 0.57. Hastelloy material was used for the first-stage and second-stage dehydration reactors. Further, the first-stage dehydration reactor and the second-stage dehydration reactor were respectively filled with a dehydration reaction catalyst.

A feed stream supply unit was provided at the upper end of the first-stage dehydration reactor, through which vaporized lactic acid molecule feed was supplied. A transfer line was provided between the first-stage dehydration reactor and the second-stage dehydration reactor, and the temperature was set so as to maintain a temperature of 390°C by a temperature regulator. The temperature regulator used was a shell and tube type heat exchanger. Further, a discharge line was provided at a lower end of the second-stage dehydration reactor to discharge the reaction product which has finished the dehydration reaction.

As the dehydration reaction catalyst, a composite calcium phosphate-based catalyst consisting of $Ca_2P_2O_7$ and $Ca_5(PO_4)_3(OH)$ formed into cylindrical pellets with a diameter of about 3 mm and a length of about 3 mm was used. At this time, the mixing weight ratio of $Ca_2P_2O_7$ and $Ca_5(PO_4)_3(OH)$ was 7:3.

A vaporized lactic acid feed stream having a temperature of 390°C was supplied to an upper end of the first-stage dehydration reactor. The residence time of the vaporized lactic acid feed stream inside the first-stage dehydration reactor was 0.1 seconds. At this time, the temperature drop in the first-stage dehydration reactor was 13.6°C, and the discharge temperature from the first-stage dehydration reactor was 376.4°C.

The stream which had finished the reaction in the first-stage dehydration reactor was transferred to the second-stage dehydration reactor through a transfer line. At this time, the temperature of the stream immediately discharged from the first-stage dehydration reactor was 376.4°C. Thus, the stream discharged from the first-stage dehydration reactor was heated in the transfer line connected to a lower end of the first-stage dehydration reactor, so that the temperature was set to be 390°C when introduced into the second-stage dehydration reactor.

At this time, the residence time of the stream transferred from the first-stage dehydration reactor inside the second-stage dehydration reactor was 1.4 second. Thereby, the value of Mathematical Formula 2 was 0.07.

The product stream which has finished the dehydration reaction in the second-stage dehydration reactor was discharged through the discharge line, and the temperature was 349.4°C.

Similarly, the product stream which had finished the entire reaction was collected from a lower end of the second-stage dehydration reactor and quantitatively analyzed by HPLC, and the acrylic acid yield and the lactic acid conversion rate were calculated. At this time, the HPLC analysis method and the method of calculating the acrylic acid yield and the lactic acid conversion rate were performed in the same manner as in Example 1.

**[Comparative Example 2]**

The two-stage dehydration reactors for a dehydration reaction were fixed bed reactors, each of which was filled with a dehydration reaction catalyst. The length of the first-stage dehydration reactor was 2.5 m and the inner diameter was 4 m. The length of the second-stage dehydration reactor was 0.5 m and the inner diameter was 4 m. Thereby, the value of Mathematical Formula 1 was 0.83. Hastelloy material was used for the first-stage and second-stage dehydration reactors. Further, the first-stage dehydration reactor and the second-stage dehydration reactor were respectively filled with a dehydration reaction catalyst.

A feed stream supply unit was provided at the upper end of the first-stage dehydration reactor, through which vaporized lactic acid molecule feed was supplied. A transfer line was provided between the first-stage dehydration reactor and the second-stage dehydration reactor, and the temperature was set so as to maintain a temperature of 390°C by a temperature regulator. The temperature regulator used was a shell and tube type heat exchanger. Further, a discharge line was provided at a lower end of the second-stage dehydration reactor to discharge the reaction product which has finished the dehydration reaction.

As the dehydration reaction catalyst, a composite calcium phosphate-based catalyst consisting of $Ca_2P_2O_7$ and $Ca_5(PO_4)_3(OH)$ formed into cylindrical pellets with a diameter of about 3 mm and a length of about 3 mm was used. At this time, the mixing weight ratio of $Ca_2P_2O_7$ and $Ca_5(PO_4)_3(OH)$ was 7:3.

A vaporized lactic acid feed stream having a temperature of 390°C was supplied to an upper end of the first-stage dehydration reactor. The residence time of the vaporized lactic acid feed stream inside the first-stage dehydration reactor was 1.3 seconds. At this time, the temperature drop in the first-stage dehydration reactor was 44.3°C, and the discharge temperature from the first-stage dehydration reactor was 345.7°C.

The stream which had finished the reaction in the first-stage dehydration reactor was transferred to the second-stage dehydration reactor through a transfer line. At this time, the temperature of the stream immediately discharged from the first-stage dehydration reactor was 345.7°C. Thus, the stream discharged from the first-stage dehydration reactor was heated in the transfer line connected to a lower end of the first-stage dehydration reactor, so that the temperature was set to be 390°C when introduced into the second-stage dehydration reactor.

At this time, the residence time of the stream transferred from the first-stage dehydration reactor inside the second-stage dehydration reactor was 0.24 second. Thereby, the value of Mathematical Formula 2 was 0.84.

The product stream which had finished the dehydration reaction in the second-stage dehydration reactor was discharged through the discharge line, and the temperature was 380.5°C.

Similarly, the product stream which has finished the entire reaction was collected from a lower end of the second-stage dehydration reactor and quantitatively analyzed by HPLC, and the acrylic acid yield and the lactic acid conversion rate were calculated. At this time, the HPLC analysis method and the method of calculating the acrylic acid yield and the lactic acid conversion rate were performed in the same manner as in Example 1.

The reaction conditions of Examples 1 to 3 and Comparative Examples 1 and 2 and the acrylic acid yield and the lactic acid conversion rate obtained from the reaction are shown in Table 1 below.

[Table 1]

|  | Comparative Example 1 | Example 1 | Example 2 | Example 3 | Comparative Example 2 |
|---|---|---|---|---|---|
| Mathematical Formula 1 (L1/(L1+L2)) | 0.07 | 0.5 | 0.33 | 0.57 | 0.83 |
| Temperature added to first-stage reactor (°C) | 390 | 390 | 390 | 390 | 390 |
| First-stage reactor dT (°C) | 13.6 | 38.4 | 38.4 | 39.9 | 44.3 |
| Temperature discharged from first-stage reactor (°C) | 376.4 | 351.6 | 356.5 | 350.1 | 345.7 |
| Temperature added to second-stage reactor (°C) | 390 | 390 | 390 | 390 | 390 |
| Second-stage reactor dT (°C) | 40.6 | 21.2 | 26.8 | 19.1 | 9.5 |
| Temperature discharged from second-stage reactor (°C) | 349.4 | 368.8 | 363.2 | 370.9 | 380.5 |
| Acrylic acid yield (mol%) | 48.6 | 53.4 | 53.5 | 53.0 | 49.1 |
| Lactic acid conversion rate (mol%) | 76.6 | 83.9 | 84.6 | 83.1 | 76.4 |

As a result of the experiments, it was confirmed that in the case of Examples satisfying Mathematical Formula 1 of the present disclosure, the target yield of 53% or more was achieved, but in the case of Comparative Examples that did not satisfy the Mathematical Formula, the yield appeared to be less than 53%.

**Experimental Example 2**

The simulation program Aspen Plus V11 and gPROCESS 2022.1.0 program was used, and the value of Mathematical Formula 1 (L1/(L1+L2)) was used as a variable, so that the acrylic acid yield (mol%) according to the value of Mathematical Formula 1 was simulated, and the results are shown in Table 2 below and FIG. 2.
The dehydration reaction conditions of the simulation program were entered by configuring with a two-stage reactor with reference to Experimental Example 1.

[Table 2]

|  | Value of Mathematical Formula 1 (L1/(L1+L2)) | | | | | | |
|---|---|---|---|---|---|---|---|
|  | 0.93 | 0.83 | 0.67 | 0.50 | 0.33 | 0.17 | 0.07 |
| Acrylic acid yield (mol%) | 45.8 | 49.1 | 52.1 | 53.4 | 53.5 | 52.1 | 48.6 |

Referring to Table 2 and FIG. 2, it was confirmed that the resulting acrylic acid yield appears in the form of a quadratic function in which the coefficient of the highest order term is a negative number, and it was also confirmed that in order to achieve the acrylic acid yield of 53 mol% or more, which is the target of the present disclosure, the value of L must satisfy 0.3 to 0.6.

**Claims**

1. An apparatus for producing acrylic acid, which comprises a two-stage reactor for lactic acid dehydration reaction, with the two-stage reactor comprising:

   a feed supply unit to which lactic acid gas is supplied;

a first-stage dehydration reactor filled with a catalyst for lactic acid dehydration reaction; and
a second-stage dehydration reactor filled with a catalyst for lactic acid dehydration reaction,
wherein the apparatus satisfies the following Mathematical Formula 1:

[Mathematical Formula 1]

$$0.3 \leq \frac{L_1}{L_1 + L_2} \leq 0.6$$

wherein in Mathematical Formula 1, $L_1$ is the length of the first-stage dehydration reactor, and $L_2$ is the length of the second-stage dehydration reactor.

2.  The apparatus according to claim 1,
    wherein a yield of acrylic acid obtained from the apparatus for producing acrylic acid is 53% or more.

3.  The apparatus according to claim 1,
    wherein the first-stage dehydration reactor and the second-stage dehydration reactor are connected by a transfer line,
    wherein the transfer line further comprises a heating unit.

4.  A method for producing acrylic acid, the method comprising:

    a first step of supplying a feed stream containing lactic acid gas molecules from a feed supply unit to an upper end of a first-stage dehydration reactor filled with a catalyst, progressing a dehydration reaction, and then discharging the stream to the lower end thereof; and
    a second step of supplying a stream discharged from the lower end of the first-stage dehydration reactor to an upper end of a second-stage dehydration reactor filled with a catalyst, progressing a dehydration reaction, and then discharging the stream to the lower end,
    wherein the feed stream supplied to the upper end of the first-stage dehydration reactor contains lactic acid gas, and
    wherein the method satisfies the following Mathematical Formula 2:

[Mathematical Formula 2]

$$0.3 \leq \frac{T_1}{T_1 + T_2} \leq 0.6$$

wherein in Mathematical Formula 2, $T_1$ is an average time that the feed stream resides in the first-stage dehydration reactor, and $T_2$ is an average time that the stream supplied to the second-stage dehydration reactor resides in the second-stage dehydration reactor.

5.  The method for producing acrylic acid according to claim 4,
    wherein the method has an acrylic acid yield of 53 mol% or more.

6.  The method for producing acrylic acid according to claim 4,
    wherein the stream discharged from the lower end of the first-stage dehydration reactor is transferred to the second-stage dehydration reactor through a transfer line, and the temperature of the stream during transfer is maintained at 370°C or more and 410°C or less.

[FIG. 1]

L = L1 + L2

[FIG. 2]

$$y = -33.411x^2 + 29.758x + 47.259$$
$$R^2 = 0.9773$$

## INTERNATIONAL SEARCH REPORT

| | International application No. |
|---|---|
| | **PCT/KR2024/019883** |

| A. | CLASSIFICATION OF SUBJECT MATTER |
|---|---|

**C07C 51/377**(2006.01)i; **C07C 57/04**(2006.01)i; **B01J 8/00**(2006.01)i; **B01J 8/02**(2006.01)i

According to International Patent Classification (IPC) or to both national classification and IPC

| B. | FIELDS SEARCHED |
|---|---|

Minimum documentation searched (classification system followed by classification symbols)

C07C 51/377(2006.01); B01J 27/18(2006.01); B01J 37/00(2006.01); C07C 51/347(2006.01); C07C 51/43(2006.01); C07C 51/44(2006.01); C07C 51/50(2006.01); C07C 57/04(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 아크릴산(acrylic acid), 젖산(lactic acid), 탈수반응(dehydration reaction), 체류시간 (duration time)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 2016-175840 A (NIPPON SHOKUBAI CO., LTD.) 06 October 2016 (2016-10-06)<br>See claim 1; paragraphs [0026]-[0028]; and example 1. | 1-6 |
| A | KR 10-2016-0122749 A (ARKEMA INC.) 24 October 2016 (2016-10-24)<br>See claims 1 and 16. | 1-6 |
| A | KR 10-2022-0052742 A (KOREA RESEARCH INSTITUTE OF CHEMICAL TECHNOLOGY) 28 April 2022 (2022-04-28)<br>See claims 1, 4, 5, 8 and 9. | 1-6 |
| A | KR 10-2023-0054198 A (LG CHEM, LTD.) 24 April 2023 (2023-04-24)<br>See claim 1. | 1-6 |
| A | KR 10-2023-0094175 A (LG CHEM, LTD.) 27 June 2023 (2023-06-27)<br>See claims 1, 5, 7 and 12. | 1-6 |

☐ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **17 March 2025** | **17 March 2025** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2024/019883**

| Patent document cited in search report | | | Publication date (day/month/year) | Patent family member(s) | | | Publication date (day/month/year) |
|---|---|---|---|---|---|---|---|
| JP | 2016-175840 | A | 06 October 2016 | WO | 2015-016217 | A1 | 05 February 2015 |
| KR | 10-2016-0122749 | A | 24 October 2016 | BR | 112016019248 | A2 | 15 August 2017 |
| | | | | BR | 112016019248 | B1 | 15 March 2022 |
| | | | | CN | 106061934 | A | 26 October 2016 |
| | | | | CN | 106061934 | B | 03 May 2019 |
| | | | | EP | 3110786 | A1 | 04 January 2017 |
| | | | | EP | 3110786 | B1 | 25 August 2021 |
| | | | | JP | 2017-509613 | A | 06 April 2017 |
| | | | | JP | 6703950 | B2 | 03 June 2020 |
| | | | | MX | 2016010692 | A | 09 May 2017 |
| | | | | SA | 516371688 | B1 | 16 April 2019 |
| | | | | SG | 11201606743 | A | 29 September 2016 |
| | | | | TW | 201534581 | A | 16 September 2015 |
| | | | | TW | I658035 | B | 01 May 2019 |
| | | | | US | 10112885 | B2 | 30 October 2018 |
| | | | | US | 2017-0174604 | A1 | 22 June 2017 |
| | | | | WO | 2015-126704 | A1 | 27 August 2015 |
| KR | 10-2022-0052742 | A | 28 April 2022 | KR | 10-2448165 | B1 | 28 September 2022 |
| KR | 10-2023-0054198 | A | 24 April 2023 | CN | 116568663 | A | 08 August 2023 |
| | | | | EP | 4249463 | A1 | 27 September 2023 |
| | | | | JP | 2023-553060 | A | 20 December 2023 |
| | | | | JP | 7584850 | B2 | 18 November 2024 |
| | | | | US | 2024-0025833 | A1 | 25 January 2024 |
| | | | | WO | 2023-063525 | A1 | 20 April 2023 |
| KR | 10-2023-0094175 | A | 27 June 2023 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230176547

- KR 1020240177830